# EUROPEAN PATENT APPLICATION

(11) **EP 3 381 498 A1**
(43) Date of publication of application: **03.10.2018**
(21) Application number: 16868205.2
(22) Date of filing: 25.11.2016
(51) Int. Cl.: A61M 37/00

(54) **MICRONEEDLE AND METHOD FOR PRODUCING SAME**

(30) Priority: 27.11.2015 JP 2015231704
(71) Applicant: Labo Juversa Co., Ltd., Chuo-ku Sapporo-shi Hokkaido 060-0001 (JP)
(72) Inventor: ONO, Ichiro, Sapporo-shi Hokkaido 060-0001 (JP)
(74) Representative: Hamer, Christopher K.
(86) International application number: PCT/JP2016/004967
(87) International publication number: WO 2017/090254

(57) **Abstract**

An object of the present invention is to solve problems of microneedles produced by conventional methods, and provide a microneedle with improved penetrability and shape retainability. A conical microneedle (10) includes a tip part (2) containing a drug and biodegradable polymer, and a base part (4) that does not contain drug, a tip (1) side is formed as a tip-reinforced region (6), a base (3) side adjacent to the tip-reinforced region with or without coating to the tip art of the microneedle(6) is formed as a non-reinforced region (7), and a hardness of the tip-reinforced region (6) is higher than the hardness of the non-reinforced region (7). A microneedle patch including the microneedle (10) according to the present invention can reduce pressing strength on application to a patient, and alleviate pain and fear to the patient.

## Description

### Technical Field

The present invention relates to a microneedle with improved penetrability and shape retainability (ability to prevent deformation) for percutaneously delivering a predetermined drug into dermis, and a method for producing the same.

### Background Art

In recent years, microneedles have been increasingly used in medical, esthetic and health care, and regenerative medicine fields. Specifically, a microneedle patch having a certain size and including a plurality of microneedles has been used to administer a target drug, vaccine, or the like, through, for example, a human body surface such as skin or mucosa into epidermis or dermis. Known methods for fabricating such microneedles include, for example, a method for filling a female mold having a plurality of recesses with a needle forming material using a squeegee and then drying and curing the material for fabricating microneedles (Patent Document 1), an inkjet method for discharging droplets of a microneedle forming material into a female mold having a plurality of recesses by computer control using jet portions for an inkjet printer and then drying and curing the material for fabricating microneedles (Patent Document 2), or the like.

Since human skin has different elasticity or hardness depending on areas or ages, a microneedle often cannot successfully penetrate the skin and cannot dispense a drug into a predetermined area. Also, for a microneedle with low penetrability, pressing strength of an applicator into the skin needs to be increased, which causes pain and fear to a patient. Thus, improvements in penetrability of a microneedle have been proposed so far. For example, a method for drying and shrinking a microneedle to curve a surface thereof inward, thereby reducing a radius of curvature of a tip part (Patent Document 3), or a microneedle including a first portion on a tip side having high hardness and a second portion on a base side having low hardness (Patent Document 4) have been proposed.

### Prior Art Documents

### Patent Documents

[Patent Document 1] Japanese unexamined Patent Application Publication No. 2012-200572
[Patent Document 2] Japanese unexamined Patent Application Publication No. 2015-136422
[Patent Document 3] Japanese unexamined Patent Application Publication No. 2008-142183
[Patent Document 4] Japanese unexamined Patent Application Publication No. 2015-016160

### Summary of the Invention

### Object to be Solved by the Invention

As described above, a plurality of proposals have been made to improve penetrability of a microneedle. However, for example, by the method described in Patent Document 3, the entire microneedle formed by increasing an amount of solvent is dried and shrunk, resulting in the reduction in the entire length of the microneedle. This may make it difficult to always complete a microneedle having a desired size and shape, and limit an effect of reducing a radius of curvature of a tip. Also, by the method described in Patent Document 4, strength of the second portion may be reduced depending on drugs contained in the microneedle, and sufficient penetrability sometimes cannot be obtained. In addition, a microneedle consisting of biodegradable polymer has a problem that a tip is melted and deformed with time, thereby significantly reducing penetrability.

An object of the present invention is to solve the problems of the microneedles produced by the conventional methods as described above, and provide a microneedle with improved penetrability and shape retainability (ability to prevent deformation).

### Means to Solve the Object

The present inventors have diligently studied to solve the object, and found that a conical microneedle including a tip part containing a drug and biodegradable polymer, and a base part containing no drug, wherein a tip side is formed as a tip-reinforced region, a base side adjacent to the tip-reinforced region is formed as a non-reinforced region, and a hardness of the tip-reinforced region is higher than the hardness of the non-reinforced region, has high penetrability and shape retainability, and completed the present invention.

Specifically, the present invention is as described below.
(1) A conical microneedle comprising a tip part containing a drug and biodegradable polymer, and a base part that does not contain drug, wherein a tip side is formed as a tip-reinforced region, and a base side adjacent to the tip-reinforced region is formed as a non-reinforced region, and a hardness of the tip-reinforced region is higher than the hardness of the non-reinforced region.
(2) The microneedle according to (1), wherein the tip-reinforced region is a region in which the tip side of the microneedle is coated with a coating material containing a curing agent.
(3) The microneedle according to (2), wherein the tip-reinforced region is a region that occupies a length of 30% to 85% of a tip side of a perpendicular line dropped from a tip to a base surface of a cone.
(4) The microneedle according to (1), wherein the tip-reinforced region is a tip side region of the tip part containing a curing agent in addition to the drug and the biodegradable polymer.
(5) The microneedle according to (1), wherein the tip-reinforced region is a tip side region of the tip part containing a curing agent in addition to the biodegradable polymer.
(6) The microneedle according to (4) or (5), wherein the tip-reinforced region is a region that occupies a length of 1% to 50% of a tip side of a perpendicular line dropped from a tip to a base surface of a cone.
(7) The microneedle according to any one of (4) to (6), wherein at least the tip-reinforced region is coated with a coating material containing a curing agent.
(8) The microneedle according to any one of (2), (3), and (7), wherein a hardness of a coating portion is higher and a friction resistance of the coating portion is lower by 5% or more as compared to before coating with the coating material.
(9) The microneedle according to any one of (2), (3), (7), and (8), wherein the coating material further contains a drug.
(10) The microneedle according to any one of (2) to (9), wherein the curing agent is one or more selected from calcium chloride, sodium chloride, polysaccharides, dextran, hyaluronic acid, chondroitin sulfate, carboxy polymer, polyacrylic acid, polylactic acid, hydroxyapatite, polyethylene glycol, fluorine compound, and silicone-based compound.
(11) The microneedle according to any one of (1) to (10), wherein a dryness of the tip-reinforced region is higher than the dryness of other portions.
(12) The microneedle according to any one of (1) to (11), wherein a radius of curvature of the tip is 5 µm or less.
(13) The microneedle according to any one of (1) to (12), wherein the hardness of the tip-reinforced region is 1.1 times or more, 1.3 times or more, or 1.5 times or more higher than hardness of the non-reinforced region.
(14) The microneedle according to any one of (4) to (13), wherein the hardness of the base part is higher than the hardness of the non-reinforced region.
(15) The microneedle according to any one of (4) to (14), wherein the hardness of the base part is equal to or lower than the hardness of the tip-reinforced region.
(16) The microneedle according to any one of (1) to (15), wherein the non-reinforced region contains a hardness reducer.
(17) A microneedle patch including a plurality of the microneedle according to any one of (1) to (16) on a substrate.
(18) A method for producing a microneedle including the following steps (A) to (D), the microneedle being a conical microneedle having a tip part and a base part and comprising a tip-reinforced region in which a tip side is coated with a coating material containing a curing agent:
   (A) filling a cone forming-original mold with a tip part forming-material containing a drug and a biodegradable polymer, and drying to form a tip part;
   (B) filling the original mold with a base part-forming material that does not contain drug, and overlaying a base part on the tip part to form a cone;
   (C) drying the cone and removing the dried cone from the original mold; and
   (D) coating a tip side of the druied cone with a coating material containing a curing agent to form a tip-reinforced region.
(19) A method for producing a microneedle including the following steps (A) to (D), the microneedle being a conical microneedle including a tip part and a base part and comprising a tip side region on the tip part containing a curing agent in addition to a biodegradable polymer:
   (A) filling a cone forming-original mold with a tip-reinforced region-forming material containing biodegradable polymer and a curing agent, and drying to form a tip-reinforced region;
   (B) filling the original mold with a non-reinforced region-forming material containing a drug and a biodegradable polymer, and overlaying a non-reinforced region on the tip-reinforced region to form a tip part;
   (C) filling the original mold with a base part-forming material that does not contain drug, and overlaying a base part on the tip part to form a cone; and
   (D) drying the cone and removing the dried cone from the original mold.
(20) The method according to (19), further including step (E) of coating a tip side of the dried cone with a coating material containing a curing agent.
(21) The method according to (19) or (20), wherein the non-reinforced refion-forming material contains a hardness reducer.
(22) The method according to any one of (18) to (21), further including step (X) of drying the tip part of the microneedle to reduce a radius of curvature.
(23) The method according to any one of (18) to (22), further including step (Y) of extending the tip part of the microneedle to reduce the radius of curvature.

### Effect of the Invention

According to the present invention, a microneedle with improved penetrability and shape retainability, and a microneedle patch including the microneedle and allowing insertion into skin with low pressing strength and drug administration can be provided, thereby allowing drug administration with low impact on a patient. Also, a microneedle patch using no special applicator can be applied, thereby extending a range of application of the microneedle patch.

### Brief Description of Drawings

[Figure 1] Figure 1 is a schematic view of a microneedle of Type I.
[Figure 2] Figure 2 is a schematic view of a microneedle of Type II.
[Figure 3] Figure 3 illustrates sections of a tip part of a microneedle.
[Figure 4] Figure 4 shows changes in total length of a microneedle after six-month refrigeration in Example 1.
[Figure 5] Figure 5 shows changes in tip diameter of the microneedle after six-month refrigeration in Example 1.
[Figure 6] Figure 6 is an enlarged photograph of a tip part of the microneedle after six-month refrigeration in Example 1.
[Figure 7] Figure 7 shows changes in total length of a microneedle immediately after production and after three-month freezing in Example 2.
[Figure 8] Figure 8 shows changes in shape of the microneedle immediately after production and after three-month freezing in Example 2.
[Figure 9] Figure 9 shows a result of comparison between residual base parts after implantation of the microneedle immediately after production and after three-month freezing in Example 2.
[Figure 10] Figure 10 shows changes in treated skin after implantation of the microneedle immediately after production and after three-month freezing in Example 2.
[Figure 11] Figure 11 shows a state before start of storage of a microneedle patch with coating in Example 3.
[Figure 12] Figure 12 shows a state after two months from start of storage of a microneedle patch without coating in Example 3, with tips being melted and deformed.
[Figure 13] Figure 13 shows a state after two months from start of storage of the microneedle patch with coating in Example 3, with shapes of tips being maintained.
[Figure 14] Figure 14 is an enlarged photograph of the state before start of storage of the microneedle patch with coating in Example 3.
[Figure 15] Figure 15 is an enlarged photograph of the state after two months from start of storage of the microneedle patch without coating in Example 3, with tips being melted and deformed.
[Figure 16] Figure 16 is an enlarged photograph of the state after two months from start of storage of the microneedle patch with coating in Example 3, with the shapes of the tips being maintained.
[Figure 17] Figure 17 shows a state of a tip part of a microneedle patch before drying in Example 4, with a radius of curvature of a tip being 20 µm or more.
[Figure 18] Figure 18 shows a state of the tip part of the microneedle patch after drying in Example 4, with the radius of curvature of the tip being improved to about 3 µm.
[Figure 19] Figure 19 shows a state after implantation of a microneedle without coating in Example 5, with an approximately 265.9 µm part from a tip being broken.
[Figure 20] Figure 20 shows a state after implantation of a microneedle with coating in Example 5, with an approximately 328.7 µm part from a tip being broken.
[Figure 21] Figure 21 shows a state of skin treated with two types of microneedle patches without coating (left) and with coating (right) after six hours in Example 5. Although the microneedle patch without coating has no trouble in implantation, it shows approximately five bleeding points. On the other hand, the microneedle patch with coating shows 50 or more bleeding points causing clearer erythema, which reveals that the microneedle patch has reached deeper dermis and a contained drug has been completely released.
[Figure 22] Figure 22 shows a state after implantation of a microneedle having a tip with a radius of curvature of 15 µm in Comparative example 1. Since the microneedle has been implanted at high implantation speed despite low sharpness of a tip part, the tip part is recessed and deformed toward a drug containing portion.
[Figure 23] Figure 23 shows a state after implantation of the microneedle having the tip with the radius of curvature of 15 µm in Comparative example 1.
[Figure 24] Figure 24 is an enlarged photograph of a tip-reinforced type microneedle in Example 6. An appearance of the tip-reinforced type microneedle is shown on the left. An appearance of a tip-reinforced type microneedle with a blue dye in place of a drug being added to a non-reinforced region is shown on the right.
[Figure 25] Figure 25 shows a breakage state of the tip-reinforced type microneedle after implantation under 9 N in Example 6.
[Figure 26] Figure 26 shows a breakage state of the tip-reinforced type microneedle after implantation under 11 N in Example 6.
[Figure 27] Figure 27 shows measurement results of residual distances from base parts of a conventional type microneedle and the tip-reinforced type microneedle in Example 6.
[Figure 28] Figure 28 is an enlarged photograph of a state of skin of a human thigh area treated with the tip-reinforced type microneedle in Example 6.
[Figure 29] Figure 29 is an enlarged photograph of a breakage state of a tip-reinforced type microneedle with coating after implantation under 9 N in Example 7.
[Figure 30] Figure 30 is an enlarged photograph of a breakage state of the tip-reinforced type microneedle with coating after implantation under 11 N in Example 7.
[Figure 31] Figure 31 shows measurement results of residual distances from base parts of a tip-reinforced type microneedle without coating and the tip-reinforced type microneedle with coating in Example 7.
[Figure 32] Figure 32 shows a state of skin of a human thigh area treated with the tip-reinforced type microneedle with coating in Example 7.

### Mode of Carrying Out the Invention

A microneedle according to the present invention may be any conical microneedle comprising a tip part containing a drug and biodegradable polymer, and a base part that does not contain drug, wherein a tip side is formed as a tip-reinforced region, a base side adjacent to the tip-reinforced region is formed as a non-reinforced region, and a hardness of the tip-reinforced region is higher than the hardness of the non-reinforced region. The hardness of the tip-reinforced region can be exemplified by preferably 1.1 times or more, more preferably 1.3 times or more, and further preferably 1.5 times or more higher than the hardness of the non-reinforced region. The hardness herein refers to a hardness value measured by a Rockwell method or a Brinell hardness test method. Hardness of the base part is preferably equal to or lower than the hardness of the tip-reinforced region and/or higher than the hardness of the non-reinforced region.

The microneedle according to the present invention may be favorably exemplified by a microneedle comprising a tip-reinforced region in which a tip side is coated with a coating material containing a curing agent (Type I), and a microneedle comprising a tip-reinforced region in which a tip side region of a tip part further contains a curing agent (Type II).

Drugs contained in the tip part of the microneedle according to the present invention may include, but not limited to, for example, antidiabetic drugs, antihypertensive agents, anticancer agent, antiallergic agents, hormones, growth factors, vaccines, analgesics, biological products, gene therapy agents, and allergen testing materials. In particular, drugs that require subdermal injection can be favorably exemplified since the microneedle can be used to administer a drug into dermis through a hole formed in skin. The content of the drug may be determined according to its type or form.

Any biodegradable polymer may be used for forming the tip part of the microneedle as long as it is degraded in body after implanted into the skin to release a drug. The biodegradable polymer may include, for example, protein such as gluten or gelatin, polysaccharides such as hyaluronic acid, chondroitin sulfate, alginic acid, starch, or dextran, polylactic acid, polyglycolic acid, lactic acid-glycolic acid copolymer, polycaprolactone, polyhydroxybutyrate, polyethylene glycol, or polypropylene glycol, and may be one polymerized with a drug to control solubility of biodegradable polymer, thereby allowing sustained release of the drug.

Materials for forming the base part of the microneedle may include biodegradable polymer selected from protein such as gluten or gelatin, polysaccharides such as hyaluronic acid, chondroitin sulfate, alginic acid, starch, or dextran, polylactic acid, polyglycolic acid, lactic acid-glycolic acid copolymer, polycaprolactone, polyhydroxybutyrate, polyethylene glycol, and polypropylene glycol, or non-biodegradable polymer selected from polyolefin such as polyethylene or polypropylene, polystyrene, polyester, polyurethane, polyamide, and fluorocarbon resin. The material for forming the base part may be the same as a material for forming the non-reinforced region except a drug. In this case, the hardness of the base part that does not contain drug is higher than that of the non-reinforced region containing a drug.

The microneedle according to the present invention may be produced using any known methods including, for example, a method for fabricating an original mold including a plurality of recesses having a shape of microneedles, and filling the original mold with a microneedle forming material using a dispenser, an inkjet device, a squeegee, or the like.

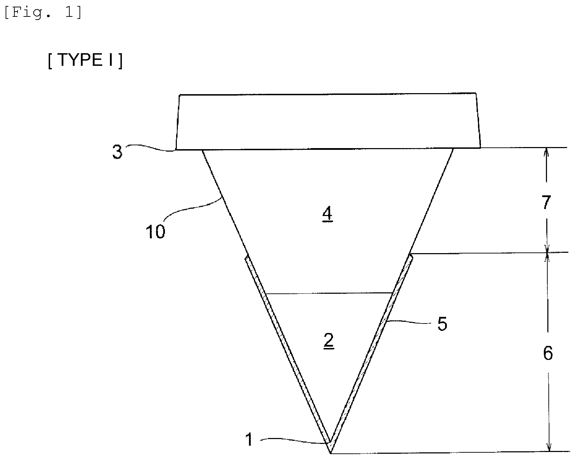

An example of the microneedle (Type I) is shown in Figure 1. A microneedle 10 comprises a tip part 2 including a tip 1, a base part 4 including a base 3, a tip-reinforced region 6 coated with a coating layer 5, and a non-reinforced region 7 not coated with the coating layer 5. The tip-reinforced region 6 covers the entire tip part 2 and a part of the base part 4 on a tip side. The tip part 2 contains a drug, while the base part 4 does not contain drug. The coating layer 5 containing a curing agent may or may not contain the drug, but preferably contains the drug. A length of the tip-reinforced region in Type I, that is, a length on a tip side of a perpendicular line dropped from the tip of a cone that forms the microneedle to a base surface can be favorably exemplified by preferably 30% to 85%, more preferably 40% to 75%, and further preferably 50% to 65% of the total length.

Although coating of the microneedle along the total length increases strength, the microneedle is not broken in the body and not placed in the body, which makes drug administration difficult. Also, when the microneedle is to be broken at a boundary between the tip part previously containing a drug and the base part that does not contain drug, coating along a length to around the boundary ensures that the microneedle is broken around the boundary between the tip part and the base part, thereby allowing a total amount of contained drug to be released in a desired area.

The coating materials can be exemplified by biodegradable polymer selected from protein such as gluten or gelatin, polysaccharides such as chondroitin sulfate, alginic acid, starch, or dextran, polylactic acid, polyglycolic acid, lactic acid-glycolic acid copolymer, polycaprolactone, polyhydroxybutyrate, polyethylene glycol, polypropylene glycol, and silicone-based compound, surfactants selected from amphoteric, anionic, cationic, and non-ionic surfactants, ethyl alcohol, saline, or water. In this case, the coating material may contain a target drug.

Coating with the coating material may be performed by any known methods exemplified by a dip coating method, an inkjet method, a spray coating method, a deposition method, or the like. A thickness of the coating may be, for example, 10 µm or less, preferably 5 µm or less, and more preferably 3 µm or less. The coating with the coating material smooths a surface of the tip part of the microneedle to reduce friction resistance on insertion into the skin, thereby improving penetrability of the microneedle. The friction resistance is preferably lower by 5% or more, more preferably 10% or more, and further preferably 20% or more as compared to before coating with the coating material.

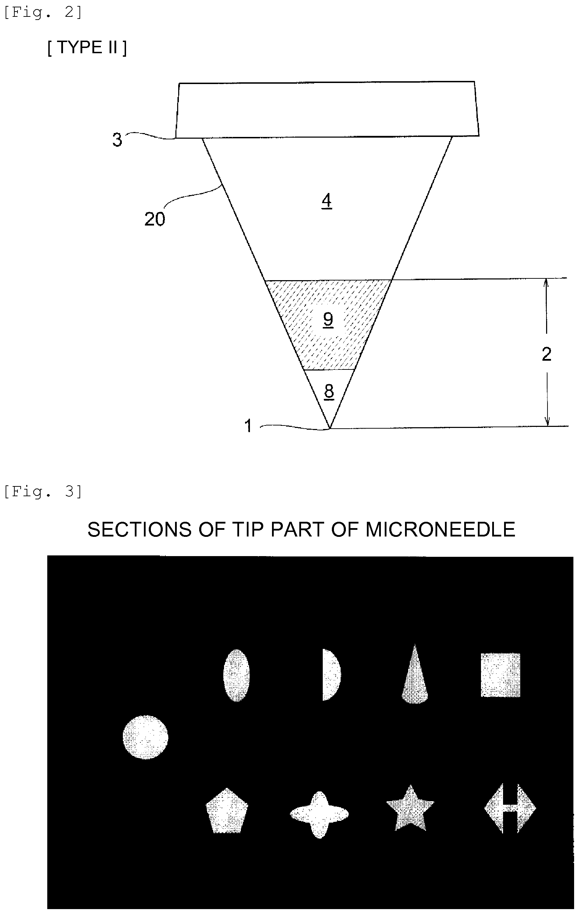

An example of the microneedle (Type II) is shown in Figure 2. A microneedle 20 comprises a tip part 2 including a tip 1, and a base part 4 including a base 3. The tip part 2 includes a tip-reinforced region 8 containing a curing agent and biodegradable polymer but containing no drug, and a non-reinforced region 9 containing a drug and an optional hardness reducer. A length of the tip-reinforced region in Type II, that is, a length on a tip side of a perpendicular line dropped from the tip of a cone that forms the microneedle to a base surface can be exemplified by preferably 1% to 50%, more preferably 3% to 40%, and further preferably 5% to 30% of the total length.

When the microneedle of Type II is fabricated by the inkjet method, the tip-reinforced region may be formed by dropping a tip-reinforced region forming material in a minimum droppable amount by an inkjet device onto an original mold. The tip-reinforced region forming material may or may not contain a drug, but preferably does not contain drug in terms of increased hardness. The coating described above may be applied to the microneedle fabricated in this manner, and a length of a coating portion is preferably 30% to 85%, more preferably 40% to 75%, and further preferably 50% to 65% of the total length of the microneedle.

The curing agent in the present invention refers to a material that can increase the hardness of the tip-reinforced region to be higher than the hardness of the non-reinforced region. The curing agent may include, for example, calcium chloride, sodium chloride, polysaccharides such as dextran, hyaluronic acid, chondroitin sulfate, carboxy polymer, polyacrylic acid, polylactic acid, hydroxyapatite, polyethylene glycol, fluorine-based compound, and silicone-based compound. The curing agent may be added to the coating material or to the tip-reinforced region forming material, and an amount of use of the curing agent may be set appropriately according to a type and intended use thereof. When the curing agent is a solid material, the material having a particle size of 5 µm or less, preferably 3 µm or less, and more preferably 1 µm or less is preferably used to smooth a surface of the tip-reinforced region. The curing agent is desirably a material that can improve shape retainability of the microneedle and maintain sharpness of the microneedle during a storage period from after production to use.

The hardness reducer in the present invention refers to a material that can reduce the hardness of the non-reinforced region to be lower than the hardness of the tip-reinforced region, and may include, for example, monosaccharides such as sucrose or dextrose, disaccharide and oligosaccharide, polylactic acid, and lactic acid.

In the present invention, to improve penetrability of the microneedle, dryness of only the tip-reinforced region may be increased. Increasing the dryness of only the tip-reinforced region can increase the hardness and reduce the radius of curvature of the tip. The tip may be drawn and extended to reduce the radius of curvature. The radius of curvature of the tip of the microneedle according to the present invention is preferably 10 µm or less, more preferably 7 µm or less, further preferably 5 µm or less, and particularly preferably 3 µm or less. The section of the tip part may be formed into, not limited to a circular shape, an elliptical shape, a triangular shape, a square shape, a plus shape, an asterisk shape, or shapes illustrated in Figure 3. The tip may be obliquely cut to increase sharpness. A helical groove may be continuously formed in an outside of the tip part of the microneedle so that the microneedle is rotated by implantation, thereby facilitating the implantation of the microneedle, and promoting breakage and separation of the microneedle from a substrate.

The total length of the microneedle according to the present invention is preferably 100 to 1000 µm, more preferably 250 to 750 µm, and further preferably 400 to 600 µm. A diameter of the base part is preferably 30 to 1000 µm, more preferably 150 to 500 µm, and further preferably 200 to 350 µm.

In the present invention, a microneedle patch refers to a patch including a plurality of the microneedles provided on a substrate. The substrate can be exemplified by a sheet consisting of biodegradable polymer selected from protein such as gluten or gelatin, polysaccharides such as chondroitin sulfate, alginic acid, starch, or dextran, polylactic acid, polyglycolic acid, lactic acid-glycolic acid copolymer, polycaprolactone, polyhydroxybutyrate, polyethylene glycol, and polypropylene glycol, or non-biodegradable polymer selected from polyolefin such as polyethylene or polypropylene, polystyrene, polyester, polyurethane, polyamide, and fluorine-based resin, paper, nonwoven fabric, or cloth. The microneedle patch according to the present invention may have an adhesive surface, and may have a structure including the substrate and the base part integrated with each other. Such a structure ensures breakage of the microneedle at a drug containing portion.

Since the microneedle according to the present invention has improved penetrability as compared to a conventional microneedle, resistance in puncture may be low even if microneedles are arranged on the microneedle patch at high density. For example, the microneedles may be arranged on the substrate at a density of 30 to 300 microneedles/cm², preferably 60 to 200 microneedles/cm², and more preferably 80 to 140 microneedles/cm². This can increase a drug containing amount per unit area as compared to a conventional microneedle patch. For the microneedle patch according to the present invention, pressing strength of an applicator into the skin can be reduced as compared to the conventional microneedle patch, thereby alleviating pain and fear on application to the skin. The pressing strength into the skin can be exemplified by preferably 11 N or less, more preferably 9 N or less, further preferably 7 N or less, and particularly preferably 5 N or less. The microneedle patch according to the present invention may be applied using a simple implantation device without any special applicator.

The microneedle patch according to the present invention is desirably stored with tips of the microneedles directed downward because if stored with the tips directed upward or sideward, the microneedle patch is significantly deformed during storage under influence of gravity. Thus, a package for storing the microneedle patch can preferably maintain the tips of the microneedles directed downward, and may be, for example, a package using a gyro function, a weight, or a magnetic force. The package may be integrated with a refrigerator. A shape of the package is not limited to a box shape, but the package may have a curved or spherical bottom surface so as to maintain the tips of the microneedles directed downward using a gyro function, a weight, a magnetic force, or the like. A label specifying the orientation for storage may be attached to the package. A storage period can be exemplified by preferably six months or more, more preferably one year or more, and further preferably two years or more which is a usual storage life of medicinal products in a refrigerator, or preferably two years or more, more preferably five years or more, and further preferably ten years or more which is a usual storage life of medicinal products in a freezer.

According to one aspect, the microneedle according to the present invention can be produced by a production method comprising the following steps (A) to (D):
(A) filling a cone forming-original mold with a tip part forming-material containing a drug and a biodegradable polymer, and drying to form a tip part;
(B) filling the original mold with a base part-forming material that does not contain drug, and overlaying a base part on the tip part to form a cone;
(C) drying the cone and removing the dried cone from the original mold; and
(D) coating a tip side of the dried cone with a coating material containing a curing agent to form a tip-reinforced region.

In step (D), the coating material containing the curing agent may be a material having high hardness and/or improving smoothness. For example, to a coating material selected from biodegradable polymer selected from protein such as gluten or gelatin, polysaccharides such as chondroitin sulfate, alginic acid, starch, or dextran, polylactic acid, polyglycolic acid, lactic acid-glycolic acid copolymer, polycaprolactone, polyhydroxybutyrate, polyethylene glycol, polypropylene glycol, and silicone-based compound, or surfactants selected from amphoteric, anionic, cationic, and non-ionic surfactants, ethyl alcohol, saline, and water, a curing agent selected from calcium chloride, sodium chloride, polysaccharides, hyaluronic acid, chondroitin sulfate, carboxy polymer, polyacrylic acid, polylactic acid, hydroxyapatite, polyethylene glycol, fluorine-based compound, and silicone-based compound may be added. The coating in step (D) is applied to the tip-reinforced region, for example, 30% to 85%, preferably 40% to 75%, and more preferably 50% to 65% of the total length of the microneedle. In one aspect, the coating in step (D) is applied to cover the tip part to a part of the base part.

In another aspect, the microneedle according to the present invention may be produced by a production method comprising the following steps (A) to (D):
(A) filling a cone forming-original mold with a tip-reinforced region-forming material containing biodegradable polymer and a curing agent, and drying to form a tip-reinforced region;
(B) filling the original mold with a non-reinforced region-forming material containing a drug and a biodegradable polymer, and overlaying a non-reinforced region on the tip-reinforced region to form a tip part;
(C) filling the original mold with a base part-forming material that does not contain drug, and overlaying a base part on the tip part to form a cone; and
(D) drying the cone and removing the dried cone from the original mold.

The production method may comprise, after steps (A) and/or (D), step (X) of drying the tip-reinforced region of the microneedle to reduce a radius of curvature. The production method may also comprise, after step (D), step (Y) of extending the tip-reinforced region of the microneedle to reduce a radius of curvature.

Further, the microneedle according to the present invention may be produced by a production method comprising the following steps (A) to (E):
(A) filling a cone forming-original mold with a tip-reinforced region-forming material containing biodegradable polymer and a curing agent, and drying to form a tip-reinforced region;
(B) filling the original mold with a non-reinforced region-forming material containing a drug and a biodegradable polymer, and overlaying a non-reinforced region on the tip-reinforced region to form a tip part;
(C) filling the original mold with a base part-forming material that does not contain drug, and overlaying a base part on the tip part to form a cone;
(D) drying the cone and removing the dried cone from the original mold; and
(E) coating a tip side of the microneedle with a coating material containing a curing agent.

The production method may comprise, after steps (A), (D) and/or (E), step (X) of drying the tip-reinforced region of the microneedle to reduce a radius of curvature. The production method may also comprise, after steps (D) and/or (E), step (Y) of extending the tip-reinforced region of the microneedle to reduce a radius of curvature.

Now, the present invention will be described more specifically with reference to examples, but a technical scope of the present invention is not limited to the examples.

### Example 1

### 1. Study on refrigeration of microneedle patch

### 1-1 Method

### 1-1-1 Fabrication of microneedle patch

A forming-material consisting of chondroitin sulfate and dextran was used to fabricate, by an inkjet method, a microneedle patch having a length of about 570 µm and containing basic fibroblast growth factor (bFGF) in a 255 µm part from a tip.

### 1-1-2 Refrigeration of microneedle patch

The microneedle patch fabricated in 1-1-1 was stored for six months in a refrigerator at 4°C with a tip part directed upward or downward. After the storage, a total length of a microneedle and a curvature of the tip part were compared with those of a microneedle patch immediately after production and studied.

### 1-2 Results

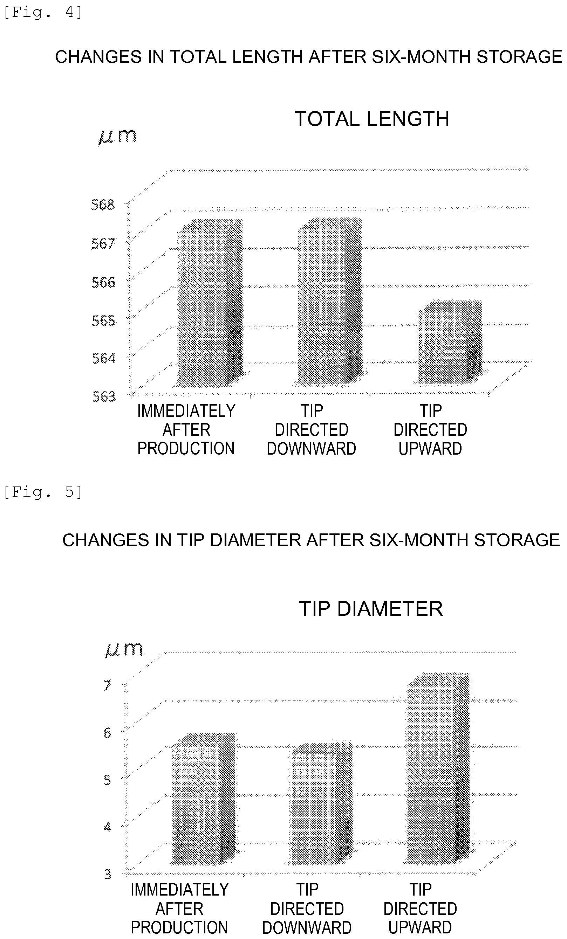

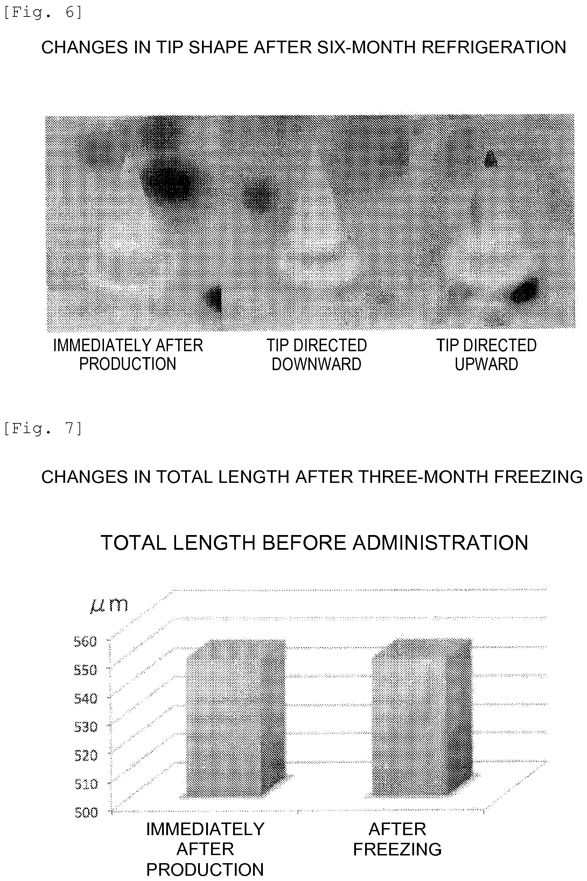

Results are shown in Figures 4 and 5. The total length immediately after production was 566.9 µm, the total length after downward storage was 567.0 µm and close to that immediately after production, but the total length after upward storage was 564.9 µm and slightly reduced. On the other hand, the curvature of the tip part immediately after production was 5.8 µm, the curvature after downward storage was 5.3 µm and slightly reduced, and the curvature after upward storage was 6.8 µm and increased. These results revealed that the downward storage has little change in shape in refrigeration. It was also confirmed from an enlarged photograph in Figure 6 that there was no difference in shape of the tip part between immediately after production and after downward storage, while the shape of the tip part was blunted after upward storage.

### Example 2

### 2. Study on freezing of microneedle patch

### 2-1 Method

### 2-1-1 Fabrication of microneedle patch

A forming-material consisting of chondroitin sulfate and dextran was used to fabricate, by an inkjet method, a microneedle patch having a length of about 548 µm and containing basic fibroblast growth factor (bFGF) in a 255 µm part from a tip.

### 2-1-2 Freezing of microneedle

The microneedle fabricated in 2-1-1 was stored for three months in a freezer at -80°C, and was compared with a microneedle immediately after production and studied in terms of shape and breakage state after implantation. Based on the results of refrigeration in 1-2 above, the microneedle was frozen with a tip directed downward in this example. The breakage state after implantation was studied by implanting the microneedles immediately after production and after freezing into skin of a human thigh area under different implantation pressures of 7 N, 9 N, 11 N, and 14 N and observing states after use.

### 2-2 Results

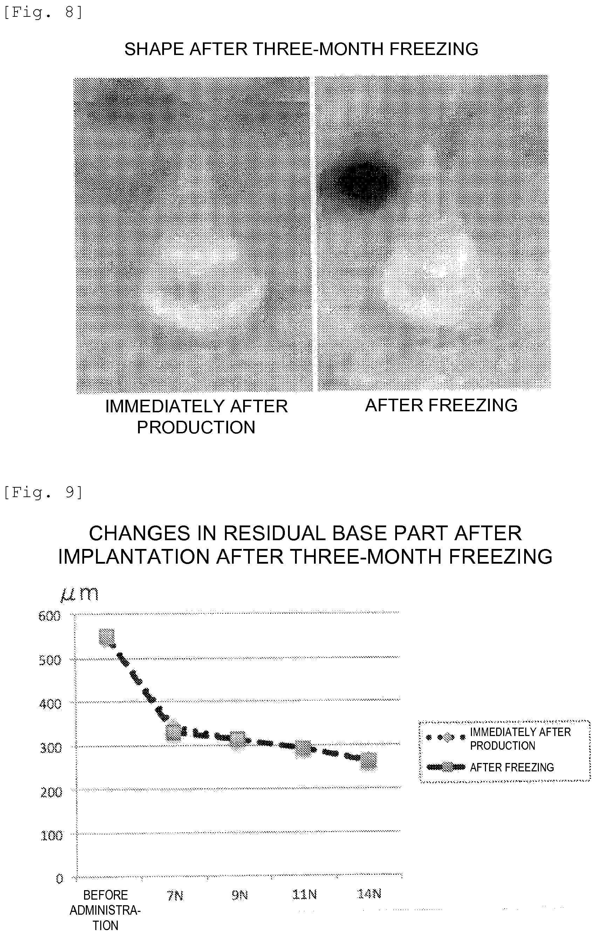

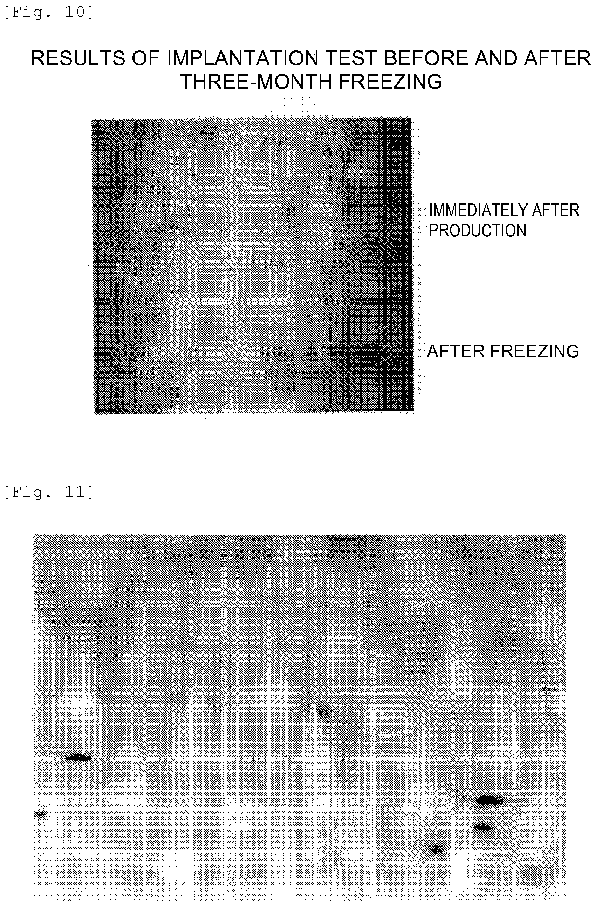

Results are shown in Figures 7 to 9. As shown in Figure 7, the total length immediately after production was 548.0 µm, and the total length after freezing was 548.0 µm and the same. As shown in an enlarged photograph in Figure 8, no significant change in the shape of the microneedle was confirmed between before and after freezing. As shown in Figure 9, the microneedles were implanted into the skin of the human thigh area under different implantation pressures of 7 N, 9 N, 11 N, and 14 N, and the results were close under all the implantation pressures. In particular, under 11 N which is pressure generally used, a residual distance from a base part of the microneedle immediately after production was 293.0 µm, and a residual distance after freezing was 291 µm and close. In this respect, it was revealed that there was no change in shape and performance after freezing. As shown in Figure 10, no difference was found in degree of appearance of erythema in an implanted area after treatment between before and after freezing.

### Example 3

### 3. Study on improvement in tip shape of microneedle patch according to the present invention

### 3-1 Method

### 3-1-1 Fabrication of microneedle patch

A forming-material consisting of chondroitin sulfate and dextran was used to fabricate, by an inkjet method, a microneedle patch having a length of 570 µm and containing basic fibroblast growth factor (bFGF) in a 255 µm part from a tip.

### 3-1-2 Coating of microneedle

A solution was formed by adding 1.2 µg of basic fibroblast growth factor (bFGF) to 0.1 ml of a solution containing a mixture of polyethylene glycol 6000 and ethyl alcohol at 1:1. The formed solution was poured into a batt to a depth of 0.3 mm, only a tip part of the microneedle patch fabricated by the method in 3-1-1 and dried was dipped in the solution and slowly pulled up after 5 seconds and dried, thereby fabricating a microneedle patch with a 300 µm part from the tip being coated with a polyethylene glycol-ethyl alcohol solution.

### 3-1-3 Evaluation of shape retainability by coating of microneedle

A microneedle with coating and a microneedle without coating were stored at room temperature for two months, and changes in tip shape with time were observed.

### 3-2 Results

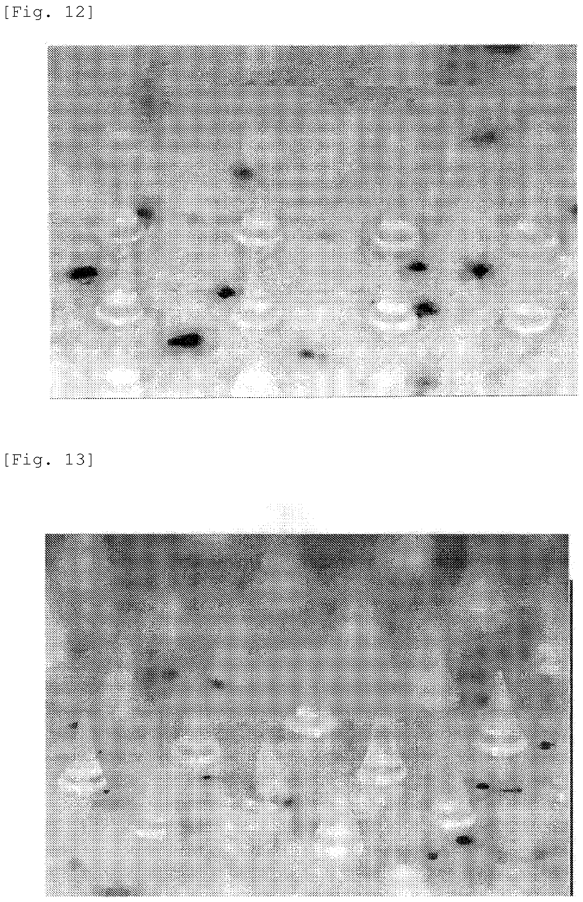

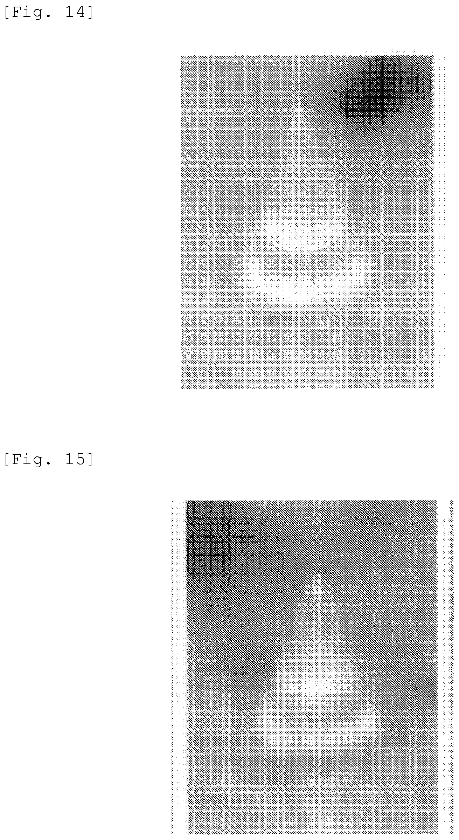

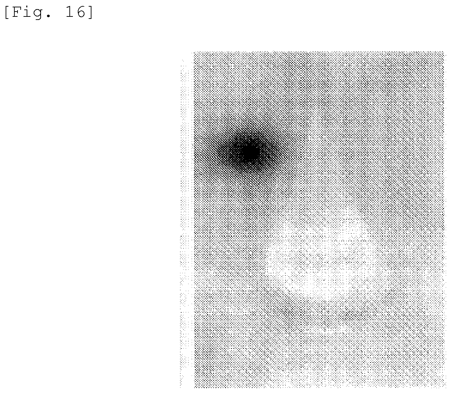

The microneedle with coating before storage is shown in Figures 11 and 14, the microneedle without coating after two-month storage at room temperature is shown in Figures 12 and 15, and the microneedle with coating after two-month storage at room temperature is shown in Figures 13 and 16. There was no change in the tip of the microneedle with coating after two-month storage at room temperature, while the tip of the microneedle without coating was melted by storage.

### Example 4

### 4. Study on improvement in radius of curvature of tip by drying of microneedle patch according to the present invention

### 4-1 Method

A solution was formed by adding 1.2 µg of basic fibroblast growth factor (bFGF) to 0.1 ml of a solution containing a mixture of polyethylene glycol 6000 and ethyl alcohol at 1:1. The formed solution was poured into a batt to a depth of 0.3 mm, only a tip part of the microneedle patch fabricated by the method in 3-1-1 and dried was dipped in the solution and slowly pulled up after 15 seconds. The microneedle patch was left in a refrigerator at 4°C with a tip of a microneedle directed downward (direction of gravity), dried for 24 hours and then returned to room temperature, and changes in tip shape were observed.

### 4-2 Results

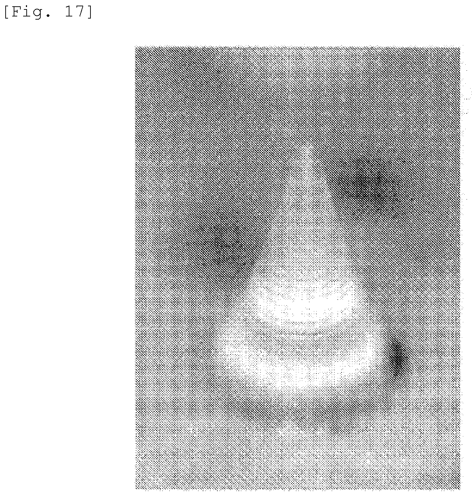

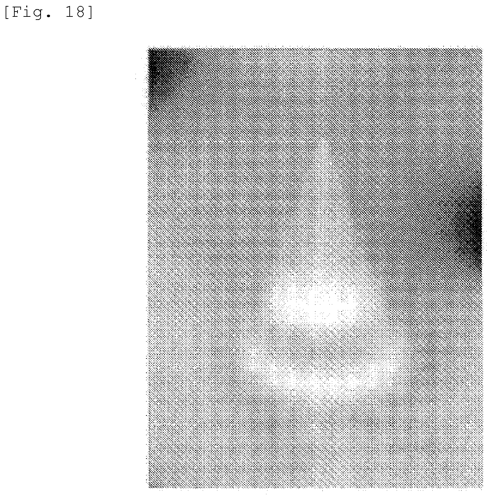

The microneedle without coating before drying is shown in Figure 17, and the microneedle with coating after drying is shown in Figure 18. A radius of curvature of the tip of the microneedle before drying was 20 µm or more, while a radius of curvature after drying was improved to 3 µm, and it was revealed that drying after coating reduces the radius of curvature of the tip.

### Example 5

### 5. Penetrability improving effect of microneedle coated by the present invention

### 5-1 Method

### 5-1-1 Fabrication of microneedle with coating

A solution was formed by adding 1.2 µg of basic fibroblast growth factor (bFGF) to 0.1 ml of a solution containing a mixture of polyethylene glycol 6000 and ethyl alcohol at 1:1. The formed solution was applied to a sterile plate to a thickness of 0.3 mm, only a tip part of the microneedle fabricated by the method in 3-1-1 and dried was dipped in the solution and slowly pulled up after 15 seconds. Then, the microneedle was dried for 24 hours in a refrigerator at 4°C, thereby fabricating the microneedle with coating of 60% of the total length. Also, a solution was formed by adding 2.4 µg of basic fibroblast growth factor (bFGF) to 0.2 ml of a solution containing a mixture of polyethylene glycol 6000 and ethyl alcohol at 1:1. The formed solution was applied to a sterile plate to a thickness of 0.6 mm, the entire microneedle fabricated by the method in 1-1-1 and dried was dipped in the solution and slowly pulled up after 15 seconds. Then, the microneedle was dried for 24 hours in a refrigerator at 4°C, thereby fabricating the microneedle with coating over the total length.

### 5-1-2 Implantation test into human skin

The microneedle patch without coating fabricated by the method in 3-1-1 and having a length of 560 µm and the microneedle patch with coating fabricated by the method in 5-1-1 were implanted into the human skin under implantation pressure of 7 N using an applicator. A state of the skin after implantation and a state of the microneedle after implantation were observed.

### 5-2 Results

### 5-2-1 State of microneedle

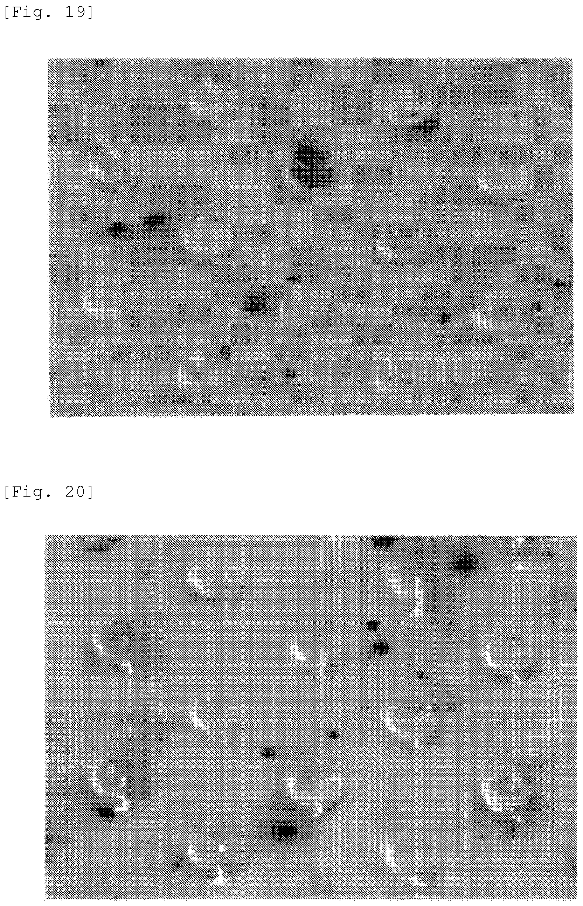

The state of the microneedle without coating after implantation is shown in Figure 19, and the state of the microneedle with 60% coating from the tip after implantation is shown in Figure 20. Breakage distances from the tips of the microneedles and breakage rates are shown in Table 1. It was confirmed that the microneedle patch with 60% coating from the tip was inserted deeper than the microneedle patch without coating (see Table 1), and the microneedle itself was broken at the base part. On the other hand, the microneedle with coating over the total length had extremely low breakability.

**TABLE 1. RESIDUAL DISTANCES FROM BASE PARTS OF MICRONEEDLES**

| GROUP N-10 | WITHOUT COATING | PEG COATING OVER 60% OF TOTAL LENGTH | PEG COATING OVER 100% OF TOTAL LENGTH |
|---|---|---|---|
| BREAKAGE DISTANCE OF MICRONEEDLE (FROM TIP) | 265.9 *µ*m | 328.7 *µ*m | 175.1 *µ*m |
| BREAKAGE RATE OF MICRONEEDLE | 73.90% | 95.8% | 32% |

### 5-2-2 State of skin

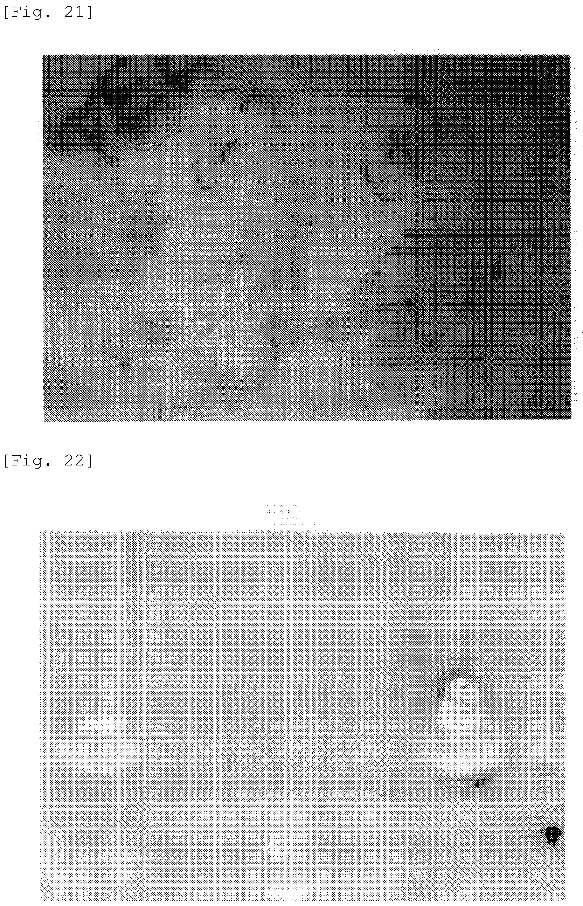

The state of skin after implantation of the microneedle is shown in Figure 21. For the microneedle without coating on the left in Figure 21, little bleeding was found in a treated area with slight erythema caused by a drug, while for the microneedle with coating on the right in Figure 21, much bleeding was found in the treated area with clear erythema caused by the drug, which was a desirable result.

### Comparative example 1

### 5'. Implantation test of microneedle having tip with large radius of curvature into skin

### 5'-1 Method

The microneedle patch having a tip with a radius of curvature of 15 µm was fabricated by the method in 3-1-1. The fabricated microneedle patch was implanted into the human skin under implantation pressure of 14 N using an applicator, and then a state of the microneedle was observed.

### 5'-2 Results

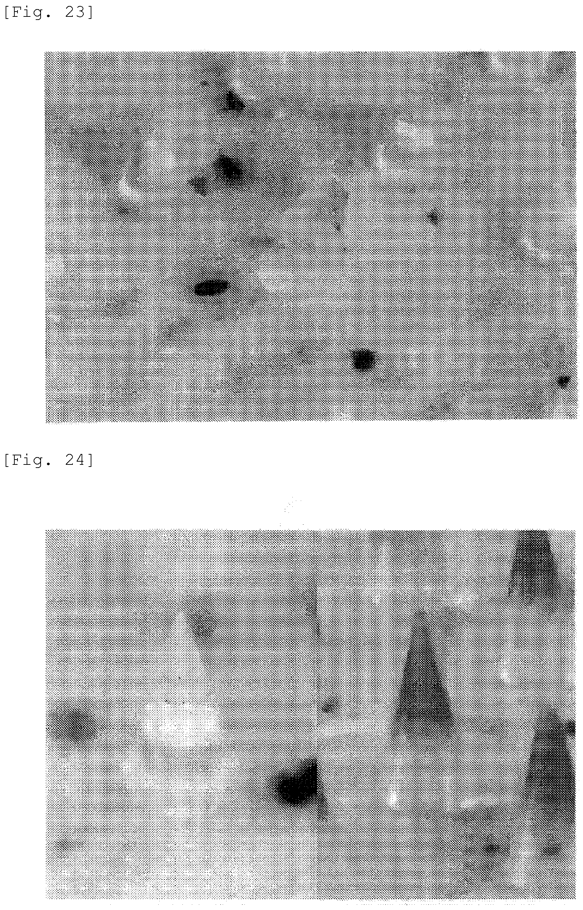

The results are shown in Figures 22 and 23. Figure 22 shows that since the microneedle has been implanted at high implantation speed despite low sharpness of the tip part, the tip part is recessed and deformed toward a drug containing portion, thereby failing in drug administration. In Figure 23, since the microneedle has been implanted at high implantation speed despite low sharpness of the tip part, the microneedle is peeled and destroyed at the base part.

### Example 6

### 6. Penetrability improving effect of tip-reinforced type microneedle

### 6-1 Method

### 6-1-1 Fabrication of tip-reinforced type microneedle patch

A microneedle having a total length of 540.0 µm was fabricated using a squeegee method, in which a 70 µm part from a tip was made of a forming-material consisting of dextran and chondroitin sulfate, a 70 µm to 255 µm part from the tip was made of a forming-material consisting of dextran and chondroitin sulfate to which basic fibroblast growth factor (bFGF) and sucrose were added, and a 255 µm part from the tip part to the base part was made of a forming material consisting of dextran and chondroitin sulfate, and the tip part that does not contain drug had hardness 1.5 times higher than that of the subsequent drug containing layer. A microneedle containing a blue dye (indigocarmine) in place of basic fibroblast growth factor (bFGF) was fabricated by the same method.

### 6-1-2 Implantation test into human skin

The two types of microneedle patches: the tip-reinforced type microneedle patch fabricated by the method in 6-1-1 and the conventional type microneedle patch fabricated by the method in 3-1-1 were implanted into the skin of the human thigh area under implantation pressures of 7 N, 9 N, 11 N, and 14 N using an applicator. A state of the skin after implantation and a state of the microneedle after implantation were observed.

### 6-2 Results

### 6-2-1 Evaluation on appearance of tip-reinforced type microneedle

An enlarged photograph of the tip-reinforced type microneedle is shown in Figure 24 (left: containing a drug, right: containing a blue dye). No difference was found in the tip-reinforced type microneedle before treatment from the conventional type microneedle in the enlarged appearance shown on the left in Figure 24. In the tip-reinforced type microneedle containing the blue dye shown on the right in Figure 24, it was apparent that a transparent tip-reinforced portion was formed at the tip, a blue drug containing portion was subsequently formed, and a transparent base part was further formed. A length of the transparent tip-reinforced region was measured and was 74.3 µm.

### 6-2-2 State of tip-reinforced type microneedle after implantation

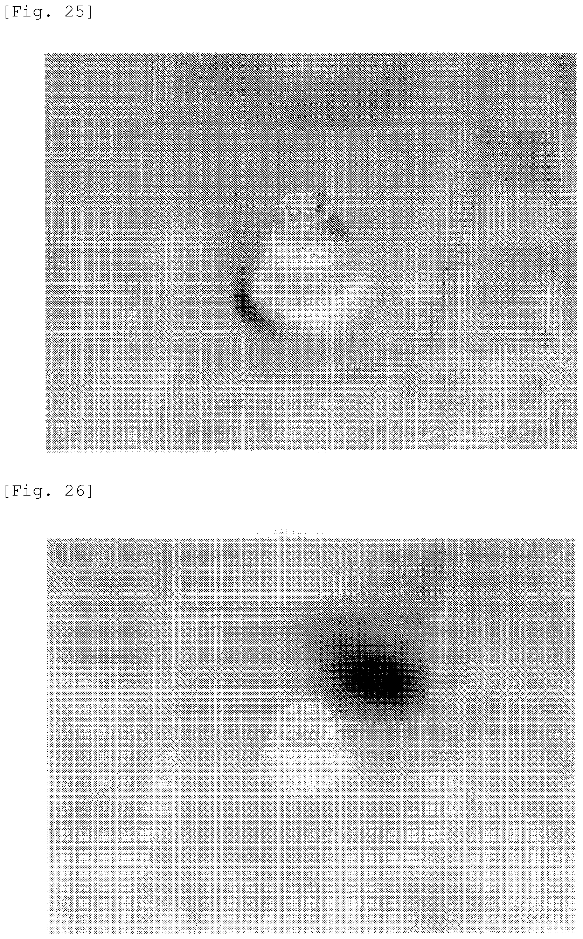

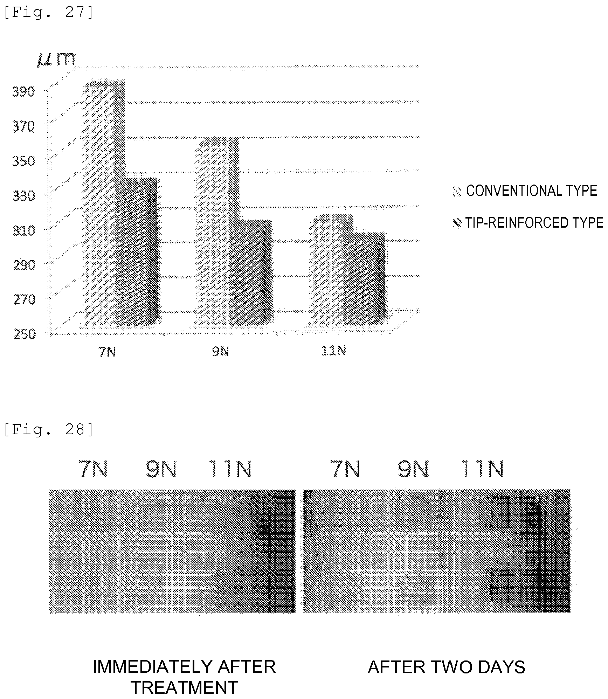

Table 2 and Figure 27 show measurement results of residual distances from the base parts of the tip-reinforced type microneedle and the conventional type microneedle after implantation into the skin. For the tip-reinforced type microneedle, the residual distance from the base part after treatment was apparently reduced as compared to that for the conventional type microneedle. Although the conventional type microneedle required implantation pressure of at least 11 N to completely implant the drug containing portion, it was revealed that the tip-reinforced type microneedle can completely implant the drug containing portion under low implantation pressure of 7 N or 9 N. Figures 25 and 26 show breakage states of the tip-reinforced type microneedle after implantation under 9 N and 11 N. It was found that the drug containing portion was reliably implanted even into the human thigh area.

**TABLE 2. RESIDUAL DISTANCES FROM BASE PARTS OF CONVENTIONAL TYPE AND TIP-REINFORCED TYPE MICRONEEDLES**

| GROUP N-10 | 7N | 9N | 11N |
|---|---|---|---|
| CONVENTIONAL TYPE | 386.8 *µ* m | 353.7 *µ* m | 309.5 *µ* m |
| TIP-REINFORCED TYPE | 330.8 *µ* m | 306.2 *µ* m | 298.6 *µ* m |

### 6-2-3 State of skin

The development of the state of the skin after implantation of the tip-reinforced type microneedle is shown in Figure 28. In the skin of the human thigh area into which the tip-reinforced type microneedle was implanted, erythema caused by a drug was found immediately after implantation (left in Figure 28). Even after two days from the treatment, clear erythema caused by the drug was found in the treated area under pressure of 11 N and also 9 N, which was a desirable result (right in Figure 28) .

### Example 7

### 7. Penetrability improving effect of tip-reinforced type microneedle with coating

### 7-1 Method

### 7-1-1 Fabrication of tip-reinforced type microneedle patch with coating

A microneedle was fabricated using a squeegee method, in which a 70 µm part from a tip was made of a forming-material consisting of dextran and chondroitin sulfate, a 70 µm to 255 µm part from the tip was made of a forming-material consisting of dextran and chondroitin sulfate to which basic fibroblast growth factor (bFGF) and sucrose were added, and a 255 µm part from the tip part to the base part was made of a forming-material consisting of dextran and chondroitin sulfate, and the tip part that does not contain drug had hardness 2 times higher than that of the subsequent drug containing layer. A solution was formed by adding 1.2 µg of basic fibroblast growth factor (bFGF) to 0.1 ml of a solution containing a mixture of polyethylene glycol 6000 and ethyl alcohol at 1:1 as a coating material. The formed solution was applied to a sterile plate to a thickness of 0.3 mm, only the tip part of the tip-reinforced type microneedle fabricated by the method described above and dried was dipped in the solution and slowly pulled up after 15 seconds. Then, the microneedle was dried for 24 hours in a refrigerator at 4°C, thereby fabricating the tip-reinforced type microneedle with 60% of the total length being coated.

### 7-1-2 Implantation test into human skin

The two types of tip-reinforced type microneedles fabricated as described above: the tip-reinforced type microneedle without coating and the tip-reinforced type microneedle with coating were implanted into the skin of the human thigh area under different implantation pressures of 7 N, 9 N, 11 N, and 14 N. A state of the skin after implantation and a state of the microneedle after implantation were observed.

### 7-2 Results

### 7-2-1 State of microneedle after implantation

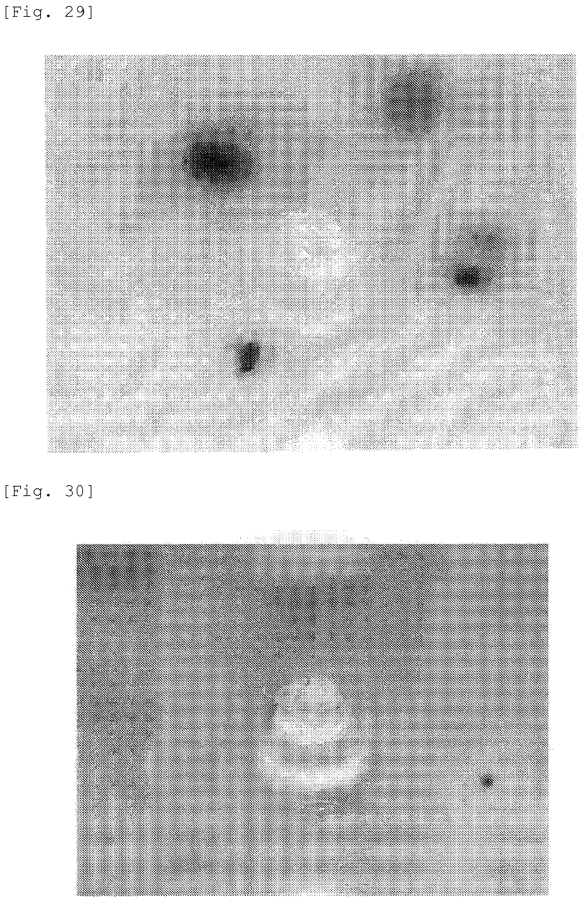

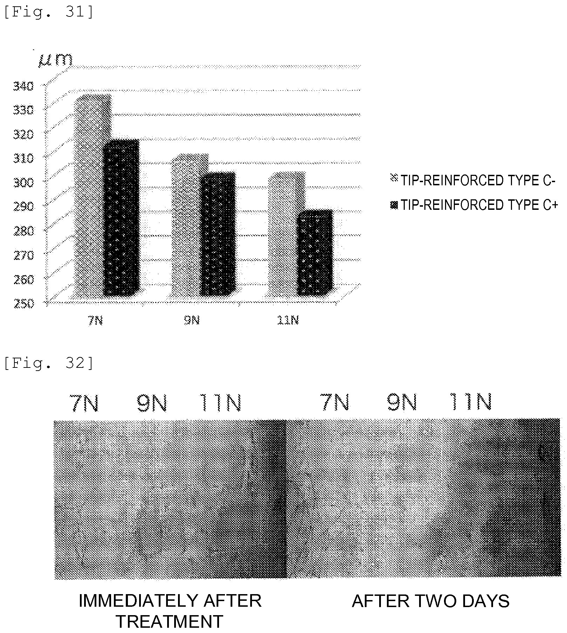

No difference was found in appearance between the tip-reinforced type microneedle without coating (Figure 24) and the tip-reinforced type microneedle with coating before treatment. Figures 29 and 30 show breakage states of the tip-reinforced type microneedle with coating after implantation under 9 N and 11 N, respectively. Both in Figures 29 and 30, the tip part of the microneedle is broken, and it is found that the drug containing portion has been reliably implanted into the human thigh area. Table 3 and Figure 31 show measurement results of residual distances from base parts of the tip-reinforced type microneedle without coating and the tip-reinforced type microneedle with coating after implantation into the skin. As shown in Table 3 and Figure 31, for the tip-reinforced type microneedle with coating, the residual distance from the base part after treatment was apparently reduced as compared to that for the tip-reinforced type microneedle without coating. Although the conventional type microneedle required implantation pressure of at least 11 N and also the tip-reinforced type microneedle without coating required implantation pressure of at least 9 N to completely implant the drug containing portion, it was revealed that the tip-reinforced type microneedle with coating can completely implant the drug containing portion even under extremely low implantation pressure of 7 N.

**TABLE 3. RESIDUAL DISTANCES FROM BASE PARTS OF TIP-REINFORCED TYPE MICRONEEDLES WITH/WITHOUT COATING**

| GROUP N-10 | 7N | 9N | 11N |
|---|---|---|---|
| TIP-REINFORCED TYPE C- | 330.8 *µ*m | 306.2 *µ*m | 298.6 *µ*m |
| TIP-REINFORCED TYPE C+ | 312.0 *µ*m | 298.6 *µ*m | 283.0 *µ*m |

| | | | |
|---|---|---|---|
| C-: WITHOUT COATING, C+: WITH COATING | | | |

### 7-2-2 State of skin

The state of the skin of the human thigh area after implantation of the tip-reinforced type microneedle with coating is shown in Figure 32. As shown on the left in Figure 32, for the tip-reinforced type microneedle with coating, even immediately after treatment under 7 N, much bleeding was found in the treated area with sufficiently clear erythema caused by the drug, which was a desirable result. Even after two days from the treatment shown on the right in Figure 32, clear erythema was found in the result under 7 N, and it was confirmed that the tip-reinforced type microneedle with coating has a treatment effect even under extremely low implantation pressure of 7 N.

### Industrial Applicability

The microneedle according to the present invention has higher penetrability and shape retainability than the conventional microneedle, and allows implantation into the patient under low pressing strength. This allows treatment with low impact on the patient as compared to the conventional microneedle, and also allows long-term storage. Thus, the microneedle according to the present invention has high industrial usability in a medical field.

### Explanation of Letters or Numerals

- 1: tip
- 2: tip part
- 3: base
- 4: base part
- 5: coating layer
- 6, 8: tip-reinforced region
- 7, 9: non-reinforced region
- 10: microneedle (Type I)
- 20: microneedle (Type II)

## Claims

1. A conical microneedle comprising a tip part containing a drug and biodegradable polymer, and a base part that does not contain drug,
wherein a tip side is formed as a tip-reinforced region, and a base side adjacent to the tip-reinforced region is formed as a non-reinforced region, and
a hardness of the tip-reinforced region is higher than the hardness of the non-reinforced region.

2. The microneedle according to claim 1, wherein the tip-reinforced region is a region in which the tip side of the microneedle is coated with a coating material containing a curing agent.

3. The microneedle according to claim 2, wherein the tip-reinforced region is a region that occupies a length of 30% to 85% of a tip side of a perpendicular line dropped from a tip to a base surface of a cone.

4. The microneedle according to claim 1, wherein the tip-reinforced region is a tip side region of the tip part containing a curing agent in addition to the drug and the biodegradable polymer.

5. The microneedle according to claim 1, wherein the tip-reinforced region is a tip side region of the tip part containing a curing agent in addition to the biodegradable polymer.

6. The microneedle according to claim 4 or 5, wherein the tip-reinforced region is a region that occupies a length of 1% to 50% of a tip side of a perpendicular line dropped from a tip to a base surface of a cone.

7. The microneedle according to any one of claims 4 to 6, wherein at least the tip-reinforced region is coated with a coating material containing a curing agent.

8. The microneedle according to any one of claims 2, 3, and 7, wherein a hardness of a coating portion is higher and a friction resistance of the coating portion is lower by 5% or more as compared to before coating with the coating material.

9. The microneedle according to any one of claims 2, 3, 7, and 8, wherein the coating material further contains a drug.

10. The microneedle according to any one of claims 2 to 9, wherein the curing agent is one or more selected from calcium chloride, sodium chloride, polysaccharides, dextran, hyaluronic acid, chondroitin sulfate, carboxy polymer, polyacrylic acid, polylactic acid, hydroxyapatite, polyethylene glycol, fluorine compound, and silicon-based compound.

11. The microneedle according to any one of claims 1 to 10, wherein a dryness of the tip-reinforced region is higher than the dryness of other portions.

12. The microneedle according to any one of claims 1 to 11, wherein a radius of curvature of the tip is 5 µm or less.

13. The microneedle according to any one of claims 1 to 12, wherein the hardness of the tip-reinforced region is 1.1 times or more, 1.3 times or more, or 1.5 times or more higher than the hardness of the non-reinforced region.

14. The microneedle according to any one of claims 4 to 13, wherein the hardness of the base part is higher than the hardness of the non-reinforced region.

15. The microneedle according to any one of claims 4 to 14, wherein the hardness of the base part is equal to or lower than the hardness of the tip-reinforced region.

16. The microneedle according to any one of claims 1 to 15, wherein the non-reinforced region contains a hardness reducer.

17. A microneedle patch comprising a plurality of the microneedle according to any one of claims 1 to 16 on a substrate.

18. A method for producing a microneedle comprising the following steps (A) to (D), the microneedle being a conical microneedle having a tip part and a base part and comprising a tip-reinforced region in which a tip side is coated with a coating material containing a curing agent:
(A) filling a cone forming-original mold with a tip part forming-material containing a drug and a biodegradable polymer, and drying to form a tip part;
(B) filling the original mold with a base part-forming material that does not contain drug, and overlaying a base part on the tip part to form a cone;
(C) drying the cone and removing the dried cone from the original mold; and
(D) coating a tip side of the dried cone with a coating material containing a curing agent to form a tip-reinforced region.

19. A method for producing a microneedle comprising the following steps (A) to (D), the microneedle being a conical microneedle including a tip part and a base part, and comprising a tip side region on the tip part containing a curing agent in addition to a biodegradable polymer:
(A) filling a cone forming-original mold with a tip-reinforced region-forming material containing biodegradable polymer and a curing agent, and drying to form a tip-reinforced region;
(B) filling the original mold with a non-reinforced region-forming material containing a drug and a biodegradable polymer, and overlaying a non-reinforced region on the tip-reinforced region to form a tip part;
(C) filling the original mold with a base part-forming material that does not contain drug, and overlaying a base part on the tip part to form a cone; and
(D) drying the cone and removing the dried cone from the original mold.

20. The method according to claim 19, further comprising step (E) of coating a tip side of the dried cone with a coating material containing a curing agent.

21. The method according to claim 19 or 20, wherein the non-reinforced region-forming material contains a hardness reducer.

22. The method according to any one of claims 18 to 21, further comprising step (X) of drying the tip part of the microneedle to reduce a radius of curvature.

23. The method according to any one of claims 18 to 22, further comprising step (Y) of extending the tip part of the microneedle to reduce the radius of curvature.
